# FASCICULE DE BREVET EUROPEEN

(11) **EP 1 237 573 B1**
(45) Date de publication et mention de la délivrance du brevet: **14.08.2013**
(21) Numéro de dépôt: 00985439.9
(22) Date de dépôt: 14.12.2000
(51) Int. Cl.: A61K 39/21, C07K 16/10, A61K 9/00, A61K 39/00, A61K 39/12

(54) **UTILISATION DE PROTEINES IMMUNOGENES IMMUNOSUPPRESSIVES OU ANGIOGENIQUES RENDUES INACTIVES POUR LA PRODUCTION D'LGA SECRETOIRES**
VEWENDUNG VON INAKTIVIERTEN IMMUNOGENEN IMMUNSUPPRESSIVEN UND/ODER ANGIOGENEN PROTEINEN ZUR SEKRETORISCHEN IGA HERSTELLUNG
USE OF INACTIVATED IMMUNOSUPPRESSIVE OR ANGIOGENIC IMMUNOGENIC PROTEINS FOR PRODUCING SECRETORY IGA'S

(30) Priorité: 15.12.1999 FR 9915825
(43) Date de publication de la demande: 11.09.2002
(73) Titulaire: NEOVACS, 75116 Paris (FR)
(72) Inventeur: ZAGURY, Daniel, F-75007 Paris (FR)
(74) Mandataire: Catherine, Alain
(86) Numéro de dépôt international: PCT/FR2000/003526
(87) Numéro de publication internationale: WO 2001/043771

(56) Documents cités:
- WO-A-96/27389
- WO-A-99/02712
- WO-A-99/39735
- GIRARD M ET AL: "New prospects for the development of a vaccine against human immunodeficiency virus type 1" COMPTES RENDUS DE L'ACADEMIE DES SCIENCES. SERIE III, SCIENCES DE LA VIE, vol. 322, no. 11, novembre 1999 (1999-11), pages 959-966, XP000946873
- BOYAKA P N ET AL: "Strategies for mucosal vaccine development." AMERICAN JOURNAL OF TROPICAL MEDICINE AND HYGIENE, vol. 60, no. 4 Supplement, avril 1999 (1999-04), pages 35-45, XP002149414 cité dans la demande
- MONTGOMERY P C ET AL: "Induction of secretory and serum antibody responses following oral administration of antigen with bioadhesive degradable starch microparticles" ORAL MICROBIOLOGY AND IMMUNOLOGY, vol. 13, no. 3, juin 1998 (1998-06), pages 139-149, XP002115802
- BOYAKA P N ET AL: "HIV Tat protein regulation of mucosal immunity." JOURNAL OF HUMAN VIROLOGY, vol. 3, no. 5, septembre 2000 (2000-09), page 257 XP000990276 2000 International Meeting of the Institute of Human Virology; Baltimore, Maryland, USA; 10-15 septembre 2000
- LE BUANEC H ET AL: "Induction in mice of anti-Tat mucosal immunity by the intranasal and oral routes.", BIOMEDICINE & PHARMACOTHERAPY = BIOMÉDECINE & PHARMACOTHÉRAPIE JUL 2001 LNKD- PUBMED:11478583, vol. 55, no. 6, July 2001 (2001-07), pages 316-320, ISSN: 0753-3322
- BORSUTZKY STEFAN ET AL: "Efficient mucosal delivery of the HIV-1 Tat protein using the synthetic lipopeptide MALP-2 as adjuvant.", EUROPEAN JOURNAL OF IMMUNOLOGY JUN 2003 LNKD- PUBMED:12778472, vol. 33, no. 6, June 2003 (2003-06), pages 1548-1556, ISSN: 0014-2980
- MARINARO MARIAROSARIA ET AL: "Mucosal delivery of the human immunodeficiency virus-1 Tat protein in mice elicits systemic neutralizing antibodies, cytotoxic T lymphocytes and mucosal IgA.", VACCINE 8 SEP 2003 LNKD- PUBMED:12922133, vol. 21, no. 25-26, 8 September 2003 (2003-09-08), pages 3972-3981, ISSN: 0264-410X
- PARTIDOS CHARALAMBOS D ET AL: "A synthetic HIV-1 Tat protein breaches the skin barrier and elicits Tat-neutralizing antibodies and cellular immunity.", EUROPEAN JOURNAL OF IMMUNOLOGY DEC 2004 LNKD- PUBMED:15549730, vol. 34, no. 12, December 2004 (2004-12), pages 3723-3731, ISSN: 0014-2980
- BORSUTZKY STEFAN ET AL: "Efficient systemic and mucosal responses against the HIV-1 Tat protein by prime/boost vaccination using the lipopeptide MALP-2 as adjuvant.", VACCINE 15 MAR 2006 LNKD- PUBMED:16406225, vol. 24, no. 12, 15 March 2006 (2006-03-15), pages 2049-2056, ISSN: 0264-410X
- FRANKEL A D ET AL: "Cellular uptake of the tat protein from human immunodeficiency virus.", CELL 23 DEC 1988 LNKD- PUBMED:2849510, vol. 55, no. 6, 23 December 1988 (1988-12-23), pages 1189-1193, ISSN: 0092-8674
- LEHNER T ET AL: "A rational basis for mucosal vaccination against HIV infection.", IMMUNOLOGICAL REVIEWS AUG 1999 LNKD- PUBMED:10566151, vol. 170, August 1999 (1999-08), pages 183-196, ISSN: 0105-2896
- LE BUANEC H ET AL: "Procedures for preparing biologically inactive, but immunogenic HIV-1 Tat protein (Tat toxoid) for human use.", BIOMEDICINE & PHARMACOTHERAPY = BIOMÉDECINE & PHARMACOTHÉRAPIE FEB 2000 LNKD- PUBMED:10721461, vol. 54, no. 1, February 2000 (2000-02), pages 41-44, ISSN: 0753-3322
- Vogel et al.: "A Compendium of Vaccine Adjuvants and Excipients (2nd edition)", pages 1-114,

## Description

La présente invention concerne l'utilisation d'une protéine Tat du ViH-1 provenant de cellules infectées par un virus ou de cellules immunitaires, d'un fragment d'une telle protéine ou encore d'une molécule d'ADN correspondant à ladite protéine ou fragment, pour la préparation d'un médicament destiné à conférer à un patient une immunité mucosale fondée sur la sécrétion d'IgA sécrétoires.

On recherche toujours des composés actifs pour lutter contre le cancer qui est le fléau médical majeur de notre époque. De nombreuses thérapies ont été développées avec un succès variable puisqu'il y a encore une mortalité importante. Ces thérapies ont d'abord été l'exérèse chirurgicale pour les tumeurs solides, la radiothérapie et la chimiothérapie. Ces thérapies semblent insuffisantes dans de nombreux cancers pour lesquels aucun succès clinique n'a été enregistré indiquant une prolongation importante de la survie des patients ou leur guérison totale.

Les cancers sont des proliférations de cellules qui peuvent ensuite essaimer dans l'organisme pour former des métastases. Il est connu que le système immunitaire d'un individu normal élimine régulièrement les cellules cancéreuses naissantes (concept d'immunosurveillance), et que la formation d'un cancer est associée 1) à l'échappement du système de surveillance immunitaire local puis à une période avancée du cancer, à une immunosuppression systémique et 2) à une prolifération des cellules endothéliales vasculaires assurant l'apport nutritif des cellules tumorales (néoangiogénèse).

La plupart des agents anticancéreux (chimiothérapie, radiothérapie) utilisés à ce jour luttent contre la réplication des cellules cancéreuses. Ces agents ne visent pas l'environnement particulier nécessaire à la prolifération des cellules cancéreuses, caractérisé à la fois par l'absence d'activité locale (paracrine) des cellules immunitaires antitumorales (échappement immunitaire) et par l'apparition d'une vascularisation intratumorale (néoangiogénèse).

C'est pourquoi, récemment, de nouvelles approches thérapeutiques ont été introduites. Certaines visent à stimuler le système immunitaire antitumeur par thérapie cellulaire ou par l'activation de gènes codant pour des protéines stimulant la réponse immunitaire (thérapie génique) ou encore en immunisant directement contre des antigènes, identifiés comme spécifiques ou associés, de la tumeur du type des M AGE (vaccination). D'autres visent à lutter contre la néoangiogénèse, en utilisant des antimétabolites détruisant les cellules endothéliales (Judah Folkman). Dans ce nouveau contexte il est à noter que De Bruijn et al., (Cancer Res. (1998) 58, pages 724-731) ont décrit l'utilisation de protéines E6 et E7 natives pour induire une réponse cellulaire cytotoxique (CTL) chez des souris pour les protéger contre l'implantation de cellules tumorales.

Or la demanderesse a découvert avec étonnement, après de longues recherches, que des facteurs immunosuppressifs ou angiogéniques à action paracrine sont induits par les tumeurs. Ces facteurs qui sont solubles peuvent, d'une part, empêcher localement les cellules du système immunitaire d'agir efficacement, même si on les stimule (immunosuppression) et peuvent, d'autre part, assurer la nutrition des cellules cancéreuses, en activant la prolifération des cellules endothéliales (angiogénèse).

Le phénomène d'échappement aux défenses immunitaires cellulaires de l'hôte par induction de leur paralysie in situ est une stratégie utilisée par de nombreux cancers et est nécessaire à leur survie. Initialement l'immunosuppression reste localisée au niveau de la tumeur, puisque l'individu est encore capable de se défendre contre les autres agressions telles que des infections. Cependant à un stade plus tardif, cette immunosuppression peut s'étendre, se généraliser, ainsi que l'attestent la dissémination de métastases et la grande vulnérabilité du cancéreux face aux infections ceci en dehors même des effets débilitants dus à la chimiothérapie ou radiothérapie. En résumé, cet échappement au contrôle du système immunitaire est dû à une paralysie du système immunitaire (immunosuppression), qui l'empêche de fonctionner normalement. Cette immunosuppression met en jeu des facteurs paralysants, qui sont produits par les cellules cancéreuses ou leur environnement. La paralysie locale des cellules du système immunitaire ou immunosuppression représente donc une arme majeure des cellules cancéreuses qui leur permet d'échapper au système immunitaire de l'hôte. Ainsi, des protéines relâchées par les cellules infectées agissent comme de véritables toxines sur les cellules immunitaires alentour, les dérèglent et bloquent in situ (de manière paracrine) les cellules du système immunitaire, protégeant les cellules infectées.

Les tumeurs malignes sont caractérisées par la présence d'une vascularisation importante, qui assure un afflux de sang nécessaire à la nutrition des cellules cancéreuses. Cette vascularisation est réalisée par l'activation des cellules endothéliales vasculaires induites au contact des cellules tumorales (néoangiogénèse). Les travaux de Judah Folkman ont récemment mis en évidence que le contrôle de la néoangiogénèse tumorale pouvait représenter une arme efficace décisive contre les cancers (Folkman J., Semin. Cancer Biol., 1992, 3 (2) : 65-71).

Dans ce contexte physiopathologique, la demanderesse a découvert, après de longues recherches, dans le cas de l'ATL, du cancer du col utérin, et du sarcome de Kaposi, trois protéines impliquées dans une immunosuppression locale au niveau de la tumeur : il s'agit de la protéine Tax de HTLV 1, de la protéine E7 du papilloma virus, et de la protéine Tat du VIH-1 dans le sarcome de Kaposi. Dans ce dernier cas, il existerait aussi comme agent étiologique un virus de l'herpès (le HHV8), qui expliquerait que le sarcome de Kaposi survient aussi chez des sujets non infectés par le VIH-1. Tat a été impliquée dans le sarcome de Kaposi, mais sans que les chercheurs n'identifient son rôle générateur d'immunosuppression locale qui favorise la génération de sarcomes de Kaposi. De manière intéressante, la demanderesse a trouvé que certaines de ces protéines immunosuppressives, telles la protéine Tat du HIV-1 et la protéine E7 de HPV (souches 16 et 18) ont également des effets activateurs sur les cellules endothéliales vasculaires.

Pour lutter contre ces facteurs immunosuppressifs et/ou angiogéniques, la demanderesse a proposé d'induire par voie générale des anticorps spécifiques dirigés contre ces facteurs extra-cellulaires. C'est ainsi qu'elle a décrit l'administration des protéines précitées par les deux voies conventionnelles d'administration des vaccins, à savoir la voie orale et la voie injectable.
Ainsi, dans une précédente demande de brevet (WO 96/27389, Zagury et al.), la demanderesse a montré comment bloquer par des anticorps spécifiques les facteurs solubles relâchés par les cellules tumorales ou infectées par un virus. Par "facteurs solubles", l'on entend des facteurs (en général des protéines) synthétisés par ces cellules et relâchés dans le milieu extracellulaire soit par transport actif soit par diffusion passive. Les facteurs extracellulaires précités peuvent agir in situ soit en inhibant la réponse cellulaire immunitaire, incluant l'activation des lymphocytes T cytolytiques (CTL), soit en perturbant le réseau cytokinique, soit en satisfaisant par la néoangiogénèse les besoins nutritifs de la tumeur. Ces facteurs extracellulaires peuvent être d'origine cellulaire (cytokines) ou, pour les cancers induits par des virus ou les maladies virales, des protéines virales, principalement des protéines de régulation, présentes dans le milieu extracellulaire.

Elle a décrit notamment des moyens permettant d'obtenir, dans le milieu circulant, des anticorps de classe IgG dirigés spécifiquement contre des facteurs délétères, par exemple immunosuppressifs et/ou angiogéniques, lesdits anticorps étant susceptibles de bloquer ces facteurs et d'en neutraliser les effets sur les cellules immunitaires et/ou endothéliales. Ces anticorps spécifiques de classe IgG ont été soit induits par immunisation active (vaccination) dirigée contre les protéines notamment les facteurs solubles préalablement identifiés, soit administrés passivement (immunisation passive). Les anticorps circulants, présents dans le milieu extracellulaire, en se combinant à ces protéines, peuvent en neutraliser les effets indésirables.

La demanderesse a identifié dans au moins trois cancers viro-induits de tels facteurs solubles : le sarcome de Kaposi (HIV-1), le cancer du col de l'utérus (HPV) et la leucémie ATL (HTLV-1 et -2) Ces 3 facteurs, tous d'origine virale, sont respectivement la protéine Tat du HIV-1, la protéine E7 du HPV et la protéine Tax du HTLV -1.

De nombreux cancers sont induits par des virus comme le HIV-1 responsable du sarcome de Kaposi et d'autres cancers, le HPV à l'origine du cancer du col de l'utérus, l'HBV et l'EBV associés, respectivement, à l'Hépatome et à la maladie de Burkitt.

La maladie SIDA (Syndrome d'Immunodéficience Acquise) due au HIV-1 et caractérisée par une immunosuppression cellulaire généralisée, peut se manifester par le sarcome de Kaposi, cancer vasculaire, ou par d'autres formes de cancer dont certaines leucémies ou lymphomes L'immunosuppression cellulaire observée dans cette maladie qui favorise l'apparition de ces cancers est induite par la protéine Tat de régulation du HIV-1, qui, si elle n'appartient pas à la structure propre du virus, est relâchée par les cellules infectées dans le milieu extracellulaire. Sous cette forme extracellulaire, la protéine Tat, agissant en véritable toxine virale, exerce un effet immunosuppressif sur les cellules immunitaires avoisinantes (Zagury D. et al.; PNAS, 1998, 95 : 3851-56). De plus, au niveau du sarcome de Kaposi, il a été montré que la protéine Tat associée aux cytokines inflammatoires (IFNα, II-1, TNFα) et au BFGF, favorisait la néoangiogénèse qui forme la tumeur (Ensoli B. J. Virol. 1993, 67 : 277-287).

En fait, de par ses propriétés immunosuppressives et angiogéniques, la protéine Tat, présente dans le milieu extracellulaire, favorise dans la maladie Sida, non seulement le développement du sarcome de Kaposi, causé par le virus HHV8, mais également d'autres cancers, incluant des lymphomes ou des leucémies.

Le cancer épithélial du col de l'utérus est provoqué par certaines souches du virus HPV (souches 16 et 18). Les cellules cancéreuses de ce cancer n'expriment que 2 protéines d'apparition précoce de ce virus, la protéine E6 et la protéine E7 qui, toutes deux, ont des effets sur les facteurs régulateurs du cycle cellulaire de la cellule cancéreuse. Par ailleurs, la protéine E7, présente dans le milieu extracellulaire, explique l'apparition chez les malades de faibles taux d'anticorps anti-E7. La protéine E7 extracellulaire, tout comme la protéine Tat extracellulaire peut agir localement comme une toxine sur les cellules stromales (cellules lymphoïdes ou cellules endothéliales) de la tumeur.

Cette protéine E7 a en effet montré expérimentalement des propriétés immunosuppressives et angiogéniques. L'immunosuppression induite par la protéine E7 a été caractérisée par l'inhibition de la prolifération des cellules T stimulées par le PPD ou le toxoïde tétanique, l'inhibition de la prolifération des cellules T stimulées par des cellules allogéniques, la surproduction d'IFNα (cytokine immunosuppressive) par les cellules présentant l'antigène (APC). Le pouvoir angiogénique de la toxine E7 sur les cultures de cellules endothéliales provenant du cordon ombilical de nouveau-nés et prétraitées par la protéine E7 a été suggéré par les observations suivantes : formation de nids cellulaires nombreux, visibles au contraste de phase ; identification par Facs de marqueurs CAM (ICAM et VCAM) au sein des cellules endothéliales et modification du cytosquelette des cellules en culture observées par immunofluorescence et altération de l'expression du monoxyde d'azote synthétase inductible par les cellules endothéliales en culture, en présence de la protéine E7. Comme on le verra dans les exemples de manière plus décisive, le pouvoir angiogénique de la toxine E7 peut être directement démontré "in vitro " par l'activation des cellules endothéliales vasculaires provenant de lignées cellulaires ou de cellules fraîches de cordon ombilical de nouveau-nés induites par la protéine E7 du HPV (souche 16).

En poursuivant ces recherches, la demanderesse s'est rendue compte que lorsqu'il s'agit d'un cancer touchant une muqueuse épithéliale tel que le cancer du col de l'utérus ou encore une infection virale touchant les muqueuses génitales, vaginales, intestinales ou rectales, il était important que la réaction immunitaire destinée à lutter contre la maladie puisse agir localement, au sein de ces muqueuses pour la bloquer à un stade précoce.

Il paraissait donc souhaitable d'induire une réaction immunitaire mucosale, générant des anticorps de classe IgA secrétoires.

L'induction d'anticorps dirigés contre les facteurs viraux extracellulaires telles les protéines Tat, E7 ou Tax, comme des toxines sur les cellules stromales lymphoïdes ou endothéliales intratumorales, implique la préparation d'immunogènes biologiquement dépourvus des effets délétères de la protéine native. De tels immunogènes ou les anticorps spécifiques qu'ils induisent peuvent grâce à leurs propriétés combattre l'immunosuppression et/ou l'angiogénèse présente (s) au sein des tumeurs et donc être utiles en tant que médicaments anticancéreux. Mais l'utilisation de molécules d'ADN permet aussi d'atteindre cet objectif.
La présente demande a principalement pour objet l'utilisation, en combinaison avec un adjuvant d'immunité mucosale, de la protéine Tat du virus HIV-1, telle que définie dans la revendication 1.
La présente demande a également pour objet l'utilisation :
- d'un produit obtenu à partir de la protéine naturelle Tat du HIV-1, qui sera modifiée par
   toute technique connue comme par voie chimique, physique (dont forme galénique) ou par ingénierie génétique, de telle sorte que ses propriétés immunosuppressives sont inactivées d'au moins 70%, de préférence d'au moins 90%, notamment d'au moins 95%, par un traitement chimique, physique et/ou par construction génétique appropriée, voire par une présentation appropriée, ou
- d'une molécule d'ADN correspondant à ladite protéine inactivée par mutation ou audit fragment inactif, représentant la version toxoïde des vaccins génétiques (vaccins à ADN).
pour l'obtention d'un médicament destiné à conférer à un patient une immunité mucosale, fondée sur la sécrétion d'anticorps de classe IgA sécrétoire.

Elle a aussi pour objet l'utilisation d'un anticorps de classe IgA sécrétoire contre une protéine immunopathogène, en particulier immunosuppressive ou angiogénique à action locale induite par une cellule Infectée par un virus, pour l'obtention d'un médicament destiné à une utilisation en tant qu'agent anti-immunosuppression locale et/ou en tant qu'agent anti-angiogénique à action locale.

Etant donné que des cancers peuvent proliférer grâce à l'immunosuppression locale évoquée ci-dessus et à l'angiogénèse, les produits ci-dessus trouvent en partie leur emploi pour l'obtention d'un médicament destiné à son utilisation en tant qu'anticancéreux.

La protéine naturelle précitée est caractérisée en ce qu'elle est une protéine initialement immunosuppressive et/ou angiogénique à action locale induite par des cellules infectées par un virus ou un fragment de cette protéine.
C'est pourquoi la présente invention a pour objet l'utilisation de la protéine Tat du HIV-1 provenant de cellules infectées par un virus ou encore d'un fragment inactif de cette protéine,
ladite protéine étant une protéine initialement immunosuppressive et/ou angiogénique à action locale dont ces propriétés sont rendues inactives d'au moins 70 %, de préférence d'au moins 90%, notamment d'au moins 95%, par un traitement physique et/ou chimique, tel qu'une formolation, une carboxamidation, une carboxyméthylation, une maléimidation ou une oxydation par barbotage d'oxygène, par recombinaison génétique ou encore par un conditionnement adjuvant, ledit traitement lui conservant la propriété d'être reconnue par des anticorps dirigés contre ladite protéine, et lui conservant des propriétés immunogènes suffisantes pour générer des anticorps neutralisant ou bloquant ladite protéine native, ou encore l'utilisation d'une molécule d'ADN correspondant à ladite protéine inactivée par mutation ou audit fragment inactif, pour l'obtention d'un médicament destiné à conférer à un patient une immunité mucosale fondée sur la sécrétion d'anticorps de classe IgA.

Par " agent anti-immunosuppression ou anti-angiogénique " l'on entend que l'agent peut avoir l'un, l'autre ou les deux effets.

Par « protéine initialement immunosuppressive et/ou angiogénique à action locale » l'on entend que la protéine native, c'est-à-dire avant inactivation, produit l'un, l'autre ou les deux effets.

Les protéines rendues inactives ou fragments inactifs de la présente invention, parfois appelés ci-après "protéines inactivées" ou "toxoïdes", ou molécules d'ADN générant la protéine initialement immunosuppressive et/ou angiogénique à action locale inactivée par mutation et de ce fait présentant le toxoïde par la vaccination ADN (version toxoïde des vaccins génétiques à base d'ADN) permettent de lutter contre les cancers par une approche complémentaire de celles de l'art antérieur et spécifique, visant la paralysie du système immunitaire et/ou encore l'angiogénèse induite(s) par les substances extracellulaires produites localement dans l'environnement des cellules cancéreuses. Cette paralysie immunitaire et/ou l'angiogénèse constituent une véritable barrière protectrice et/ou une source nutritive pour la tumeur.

Les protéines rendues inactives, fragments ou molécules d'ADN selon la présente invention permettent de lutter en premier lieu contre ces facteurs protéiques immunosuppressifs et/ou angiogéniques, par formation mucosale d'anticorps de classe IgA contre ces protéines et notamment contre ces facteurs protéiques solubles pour permettre au système immunitaire d'agir efficacement et/ou pour bloquer la néoangiogénèse donc au lieu même d'entrée de l'infection ou du siège de la maladie.

Il est important d'utiliser le facteur protéique extracellulaire délétère sous une forme notamment physiquement, chimiquement et/ou génétiquement modifiée (inactivée) et non native (ou naturelle) afin qu'il n'exerce plus ses effets nocifs (paralysie paracrine du système immunitaire ou angiogénèse locale).

Les traitements physiques peuvent être réalisés par la chaleur, les radiations U.V., les rayons X ou le contact avec une atmosphère riche en O₂. Ces traitements physiques générant des modifications intramoléculaires entre radicaux chimiques (groupement thiols par exemple), peuvent de manière appropriée changer la conformation de la molécule, l'inactiver fonctionnellement tout en conservant ses propriétés immunogènes.

Le traitement chimique peut être effectué à l'aide d'un agent de couplage tel qu'un dialdéhyde, ou d'une protéine porteuse activée par un pré-traitement à l'aide d'un dialdéhyde, de préférence ou le glutaraldéhyde. Le traitement chimique peut se faire par utilisation d'un monoaldéhyde, notamment de formaldéhyde. On peut à cet égard se reporter à l'enseignement de WO-A-96/27389.

Le traitement chimique peut se faire notamment par d'autres procédés telle la carboxyméthylation ou la carboxamidation. Un exemple de technique de carboxyméthylation est illustré dans WO-A-99/33872. Le traitement chimique peut se faire également par N éthylmaléidation associée ou non à une glutaraldéhydation.

On peut encore citer comme technique d'inactivation la réaction d'au moins une fonction thiol de la protéine avec le 4-chloro-7-sulfobenzofurazane d'ammonium, le N-[iodoéthyl]-trifluoroacétamide ou le N-(6-[7-amino-4-méthylcoumarin-3-acétamido]hexyl)-3'-(2'-pyridyldithio) propionamide ainsi que la réaction d'au moins fonction amino de la protéine avec l'éthylacétimidate, un anhydride, le 2-iminothiolane chlorhydrate, le N-succinimidyl S-acétylthioacétate, le sulfosuccinimidyl acétate, le sulfosuccinimidyl-4-O-[4,4'-diméthoxytrityl]butyrate, le succinimidyl 7-amino-4-méthylcoumarin-3-acétate, le sulfosuccinimidyl 7-amino-4-méthylcoumarin-3-acétate ou le phénylglyoxal.

L'immunogène peut être inactivé grâce à une présentation galénique au sein d'un liquide huileux, tel l'adjuvant incomplet de Freund ou encore susceptible de modifier les liaisons non covalentes (forces électrostatiques, forces de Van der Waals ou liaisons hydrogène) nécessaires à ses effets toxiques.

Les modifications génétiques peuvent être obtenues par ingénierie génétique opérant des insertions, des délétions ou des substitutions de résidus, opérations destinées à diminuer ou supprimer les sites fonctionnels délétères de la molécule naturelle. Les mutants génétiques pourront ou non subir un traitement chimique et/ou physique complémentaire. Les protéines modifiées ci-dessus peuvent par exemple être préparées à partir d'une protéine ayant une séquence identique ou similaire à une séquence peptidique d'une protéine immunopathogène, en particulier immunosuppressive ou angiogénique, telle que la protéine Tat du HIV-1, ou d'un fragment de cette protéine et être obtenues par exemple par synthèse peptidique conventionnelle sur résine ou par génie génétique. Tous ces procédés sont bien connus de l'état de la technique. Les mutants inactifs mais immunogènes ont au moins une molécule d'ADN qui code pour leur production. De telles molécules d'ADN présentent un intérêt particulier dans la présente invention comme on le verra ci-après.

Afin de vérifier que la protéine native immunosuppressive et/ou angiogénique est bien reconnue par des anticorps dirigés contre ladite protéine immunosuppressive modifiée ou son fragment modifié ou non selon l'invention, on peut par exemple vérifier immunologiquement par Elisa en présence d'anticorps spécifiques, la formation de complexes antigène-anticorps.

Dans des conditions préférentielles de mise en oeuvre, le composé immunogène provient d'un composé natif (protéine ou fragment polypeptidique) traité par un aldéhyde, ou carboxamidé, ou carboxyméthylé, ou maléimidé.

Afin de déterminer si les propriétés immunogènes de la protéine modifiée immunosuppressive et/ou angiogénique ou d'un fragment de cette protéine ont été suffisamment conservées (c'est à dire si il ou elle a été inactivé(e) mais pas dénaturé) pour créer des anticorps bloquant les effets de ladite protéine native, on peut par exemple immuniser des mammifères (lapins, rats, souris) à l'aide d'un composé immunogène selon l'invention et vérifier que les anticorps produits neutralisent les activités immunosuppressives ou angiogénique de la protéine, comme on le verra pour la protéine Tat du VIH-1, la protéine E7 du papilloma virus et la protéine Tax de HTLV1 dans la partie expérimentale.

Afin de déterminer si la protéine immunosuppressive modifiée ou le fragment a perdu au moins la proportion désirée de ses propriétés immunosuppressives, on peut par exemple étudier l'effet de la protéine immunosuppressive sur l'immunosuppression des cellules mononucléées du sang périphérique humain (PBMC).

L'ADN (plasmide avec promoteur) peut être délivré aux surfaces mucosales sous forme d'ADN nu ou formulé, par exemple sous forme de liposomes cationiques ou concentré autour de particules d'or ou encore sous forme de microsphères. Il est avantageusement mis en oeuvre en présence d'adjuvants notamment des toxines bactériennes telles que CT (cholera toxine) ou de LT (entérotoxine labile d'E. coli). De telles techniques d'immunisation mucosale avec des vaccins à base de molécules d'ADN sont décrites notamment dans Microbes and Infection, 1999, 685-698 par McCluskie et al.

La protéine immunosuppressive ou angiogénique modifiée et immunogène peut dériver d'une protéine notamment immunosuppressive à action locale induite chez le malade du Sida ; on retient particulièrement la protéine Tat du virus HIV-1.

Par "dérivent" ou "dériver" d'une protéine immunopathogène, en particulier immunosuppressive ou angiogénique à action locale produite par des cellules cancéreuses ou infectées par un virus ou produite par des cellules immunitaires, l'on entend que le composé immunogène peut être constitué de la totalité ou d'un fragment de la protéine immunopathogène, en particulier immunosuppressive ou angiogénique de départ.

Il peut comporter une ou plusieurs modifications dans les acides aminés de cette protéine ou fragment telles que des délétions, substitutions, additions, ou fonctionnalisations telles qu'acylation d'acides aminés, dans la mesure où ces modifications restent dans le cadre précisé ci-dessus (absence de toxicité, caractères immunologiques). Par exemple, en général le remplacement d'un résidu leucine par un résidu isoleucine ne modifie pas de telles propriétés; les modifications doivent généralement concerner moins de 40% d'acides aminés, de préférence moins de 20% et tout particulièrement moins de 10% de la protéine immunopathogène, en particulier immunosuppressive ou angiogénique. Il est important que la protéine ou fragment modifié ne soit pas dénaturé comme on peut le faire par exemple par un traitement physique comme la chaleur afin de préserver ses sites conformationnels pour que les anticorps induits par les dérivés modifiés soient actifs vis à vis de la protéine native.

Dans des conditions préférentielles, les composés immunogènes de l'invention comportent au moins 50% de la totalité ou d'un segment de la protéine immunopathogène, en particulier immunosuppressive ou angiogénique, de préférence au moins 70%, particulièrement au moins 90%, et tout particulièrement la totalité ou quasi-totalité de ladite protéine immunosuppressive ou angiogénique.

De manière générale, en ce qui concerne les modifications, l'homologie ou la similitude entre l'immunogène modifié et la protéine ou partie de protéine immunosuppressive native, ainsi que les dimensions du composé immunogène, de même que les modalités d'utilisation, ou de couplage du composé immunogène selon l'invention à une protéine immunogène telle que le toxoïde tétanique, on peut en particulier se référer à WO-A-86/06 414 ou à EP-A-0.220.273 ou encore à PCT/US.86/00831, équivalents, dont l'enseignement est incorporé ici par référence.

On préfère aussi un composé immunogène tel que défini ci-dessus qui est un produit obtenu par recombinaison génétique présentant une homologie peptidique de 70 % au moins avec la protéine Tat du HIV-1 ou avec un segment de cette protéine.

On préfère également un composé immunogène tel que défini ci-dessus caractérisé en ce qu'il est traité par un aldéhyde, qu'il est carboxamidé, carboxyméthylé ou maléimidé.

On préfère enfin un composé immunogène tel que défini ci-dessus caractérisé par un conditionnement adjuvant qui le rend biologiquement inactif, telle qu'une émulsion huileuse en adjuvant de Freund incomplet (IFA).

On peut aussi faire dériver d'un mutant homologue le composé immunogène désiré.

Rappelons ici que par un conditionnement galénique d'une protéine active physiologiquement, on peut masquer son activité biologique tout en conservant son immunogénicité.

La réaction de carboxyméthylation permet de modifier les groupements thiols (groupements sulfhydryl) présents au niveau des résidus cystéines de l'enchaînement en acides aminés. La carboxyméthylation inactive certaines fonctions toxiques dépendantes des groupements SH comme l'a rapporté pour la protéine Tat Frankel et al. Cell Vol 55 (1988).

Mise à part la carboxyméthylation, la carboxamidation ou la maléimidation peuvent être utilisées pour bloquer les groupements SH et former des complexes S-carboxyméthylés, S-carboxamidés ou S-maléimidés.

Par exemple, la protéine Tat possède 7 cystéines. Ces cystéines participent à la formation de ponts disulfure inter et intra-chaînes et contribuent à la formation d'oligomères.

Le produit de la réaction est dans chaque cas un résidu S-carboxyméthylcystéinyl ou S-carboxyméthylamidocystéinyl.

Un fragment peut comporter de 8 à 110 acides aminés par exemple, de préférence de 12 à 60 acides aminés et notamment de 12 à 40 acides aminés. Un tel fragment peut comporter aussi du ou des côtés C ou N terminal de 1 à 5 acides aminés supplémentaires c'est-à-dire différents du segment d'origine. Un fragment doit en outre comporter une cystéine au moins pour pouvoir faire l'objet par exemple d'une carboxyméthylation. Les fragments, s'ils seront de préférence choisis inactifs par eux- mêmes, pourront en effet soumis si désiré aux mêmes traitements d'inactivation que les protéines entières ou presque entières.

La réaction de carboxyméthylation ci-dessus peut aussi être réalisée avec d'autres agents chimiques comme l'acide performique, l'acide 3-bromopropionique, l'éthylénéimine, le bromure de (2-bromoéthyl) triméthylammonium, le 2-bromoéthane sulfonate, la 1,3-propanesulfone etc....

Dans des conditions préférentielles de mise en oeuvre du procédé ci-dessus décrit, ladite protéine ou ledit fragment de départ peut se présenter sous forme fusionnée à un marqueur (FP) ou non fusionnée (P). La forme FP peut modifier per se la conformation moléculaire et par là modifier son activité.

Les protéines ou fragments de départ du procédé sont des produits connus dont des procédés d'inactivation peuvent avoir été décrits dans la littérature comme dans WO-A-99/33872. Ces protéines de départ peuvent même être commercialisées (Immunodiagnostics Inc., Cat # 1002-2) ou peuvent être préparées de manière conventionnelle.

On peut en particulier préparer les protéines ou fragments de départ ci-dessus par :
1) Synthèse par génie génétique ou par synthèse biochimique ;
2) Purification

Par génie génétique, on peut purifier les protéines produites par chromatographie d'affinité en utilisant par exemple des anticorps dirigés contre la protéine ou un de ses fragments ; on peut aussi synthétiser la protéine fusionnée à un marqueur (FP) qui servira à l'accrochage à une colonne d'affinité.

Dans d'autres conditions préférentielles de mise en oeuvre du procédé ci-dessus décrit, lorsque la protéine ou fragment est fusionné à un marqueur (FP), il est soumis à :
- une concentration, par exemple, par ultrafiltration ;
- un désalage, par exemple, par gel filtration ;
- un traitement au bromure de cyanogène ou l'entérokinase pour cliver la protéine de fusion et libérer ainsi la protéine ou fragment ;
- une concentration et diafiltration ;
- une chromatographie par échange cationique ;
- une concentration par ultrafiltration, suivie par une filtration sur gel d'exclusion.

La réaction au bromure de cyanogène ci-dessus permet de cliver les thioéthers. L'action du bromure de cyanogène sur les molécules polypeptidiques est sélective en effectuant un clivage au niveau des résidus méthionine existants. Cette réaction aboutit à la formation de 2 fragments polypeptides par résidu méthionine. Cette réaction peut être avantageusement couplée notamment avec la réaction de carboxyméthylation précédemment décrite mais elle n'est pas nécessaire à l'inactivation.

Dans d'autres conditions préférentielles de mise en oeuvre du procédé ci-dessus décrit, on prépare la protéine ou le fragment attendu(e) couplé(e) à un composé permettant sa purification, par exemple à un fragment peptidique contenant plusieurs histidines, de préférence en séquence continue de 4, 5, notamment 6 histidines ou plus permettant la fixation à une colonne de Nickel. Dans la mesure où la présence de ce composé n'induit pas de toxicité et ne modifie pas défavorablement l'immunogénicité de la protéine ou de fragment, il n'est pas nécessaire de le cliver après purification. Toutefois, dans des conditions de réalisation préférée, on clive ce composé pour l'éliminer.

La présente demande a encore pour objet l'utilisation
- d'un produit obtenu à partir de la protéine Tat du HIV-1, qui sera modifiée par
   toute technique connue comme par voie chimique, physique (dont forme galénique) ou par ingénierie génétique, de telle sorte que ses propriétés immunosuppressives sont inactivées d'au moins 70%, de préférence d'au moins 90%, notamment d'au moins 95%, par un traitement chimique, physique et/ou par construction génétique appropriée, voire par une présentation appropriée, ou
- d'une molécule d'ADN correspondant à ladite protéine inactivée par mutation ou audit fragment inactif,
pour l'obtention d'une composition pharmaceutique pour la voie muqueuse, notamment pour la voie mucosale comme orale ou pour la voie intranasale destinée à conférer à un patient une immunité mucosale, fondée sur la sécrétion d'anticorps de classe IgA sécrétoire.

La présente demande a encore notamment pour objet l'utilisation de tels produits pour la fabrication d'un traitement par la voie muqueuse pour lutter contre les protéines immunosuppressives et/ou angiogéniques à action locale induite notamment chez le malade du Sida.

Les protéines modifiées qui sont des protéines initialement notamment immunosuppressives et/ou angiogéniques à action locale induites par les tumeurs cancéreuses chez le malade du Sida et dont les propriétés immunosuppressives sont inactivées par un traitement approprié, les fragments et les molécules d'ADN correspondant à ces protéines natives inactivées par mutation ou fragments, possèdent de très intéressantes propriétés pharmacologiques. Ils sont doués notamment de remarquables propriétés antagonistes des propriétés des protéines immunosuppressives et/ou angiogéniques à action locale induites par une tumeur cancéreuse par production d'anticorps sécrétoires de classe IgA .

Ces propriétés sont illustrées ci-après dans la partie expérimentale. Elles justifient l'utilisation des protéines modifiées ci-dessus décrites, fragments et molécules d'ADN à titre de médicament.

En effet, les composés selon l'invention ont perdu leurs propriétés immunosuppressives ou leurs propriétés angiogéniques et peuvent donc être administrés chez l'homme comme on le verra ci-après dans la partie expérimentale.

C'est pourquoi la présente demande a encore pour objet une composition pharmaceutique pour la voie muqueuse, notamment pour la voie oromuqueuse comme la voie intranasale ou orale renfermant à titre de principe actif un produit obtenu à partir d'une protéine naturelle, la protéine Tat du HIV-1 selon l'invention, qui sera modifiée par toute technique connue comme par voie chimique, physique (dont forme galénique) ou par ingénierie génétique, de telle sorte que ses propriétés immunosuppressives sont inactivées d'au moins 70%, de préférence d'au moins 90%, notamment d'au moins 95%, par un traitement chimique, physique et/ou par construction génétique appropriée, voire par une présentation appropriée, ou une molécule d'ADN correspondant à ladite protéine inactivée par mutation ou audit fragment inactif.

Les médicaments selon la présente invention trouvent leur emploi par exemple dans le traitement tant curatif que des cancers, notamment des cancers induits par des virus comme, par exemple, l'ATL (Acute T cell leukemia) causé par le HTLV 1, ou le cancer du col utérin causé par le papilloma virus, ou encore le lymphome de Burkitt ou le sarcome de Kaposi causés par des virus de la famille herpès, respectivement l'Epstein-Barr (EBV) et le HHV8 ainsi que dans le traitement du SIDA.

Les composés immunogènes selon l'invention peuvent être utilisés comme suit :
On administre à un patient sous une forme adaptée à l'administration mucosale, un composé immunogène ou une molécule d'ADN selon la présente invention, par exemple par voie intranasale, en quantité suffisante pour être efficace sur le plan thérapeutique, à un sujet ayant besoin d'un tel traitement. La dose administrée peut aller par exemple de 10 à 1000 µg par voie intranasale, une fois par semaine pendant deux mois, puis périodiquement en fonction du taux des anticorps sécrétoires induits, par exemple tous les 2-6 mois.
On pourra administrer dans une même préparation deux ou plusieurs molécules immunogènes et/ou molécules d'ADN différentes pour induire des anticorps neutralisant tous les sites fonctionnels délétères au cas où une seule molécule ne porte pas tous les sites actifs de la toxine ou de la cytokine surproduite que l'on veut neutraliser.

L'invention a aussi pour objet les compositions pharmaceutiques destinées aux muqueuses qui renferment au moins un composé immunogène ou molécule d'ADN précité, à titre de principe actif.

A titre de médicaments, les composés immunogènes ou molécules d'ADN de l'invention peuvent être incorporés dans des compositions pharmaceutiques destinées à la voie muqueuse, notamment oro-muqueuse, en particulier la voie intranasale et la voie orale. L'administration peut avoir lieu en dose unique ou répétée une ou plusieurs fois après un certain intervalle de temps.

C'est pourquoi la présente demande a également pour objet une composition pharmaceutique, curative ou préventive pour la voie muqueuse, caractérisée en ce qu'elle comprend à titre de principe actif, un ou plusieurs composés immunogènes tels que définis ci-dessus, ou ses fragments ou molécules d'ADN correspondant à la protéine native à combattre. Le composé immunogène, fragment ou molécule d'ADN peut être conditionné seul ou mélangé à un excipient ou mélange d'excipients pharmaceutiquement acceptables tel qu'un adjuvant. Parmi les excipients destinés à la voie intranasale ou orale, on retient particulièrement les capryl caproyl macrogol glycérides comme le Labrasol^{®} de la société GATTEFOSSE ou l'hydroxyde d'alumine (Alhydragel, Superfos, Danemark).

Il est à noter qu'administré tel quel, selon une formulation orale conventionnelle, le principe actif selon l'invention serait inactif.

Pour l'administration orale selon l'invention, on associera le principe actif à un adjuvant d'immunité mucosale tel qu'un mutant de CT ou de LT.

On retient tout particulièrement les formes galéniques décrites par Boyaka et al : « Strategies for mucosal vaccine development » dans Am. J. Trop. Med. Hyg. 60(4), 1999, pages.35-45. On peut citer aussi les microgranules gastro résistants, notamment bioadhésifs tels que eux décrits par Rojas et al dans Pharmaceutical Research, Vol. 16, N° 2, 1999, page 255.

La présente demande a plus particulièrement pour objet un vaccin mucosal contenant à titre d'immunogène, un composé immunogène défini ci-dessus et notamment une protéine initialement immunosuppressive et/ou angiogénique à action locale induite par une tumeur cancéreuse ou par des cellules infectées par un virus tel le VIH ou un fragment de cette protéine dont les propriétés immunosuppressives et/ou angiogéniques sont inactivées d'au moins 70 % par un traitement approprié ou une molécule d'ADN correspondant à cette protéine inactivée par mutation ou audit fragment inactif.

Dans des conditions préférentielles de mise en oeuvre, on retient une composition pharmaceutique vaccinale ci-dessus, caractérisée en ce qu'elle comprend un adjuvant d'immunité mucosale, tel un mutant de CT (cholera toxine) ou de LT (entérotoxine labile d'E. coli).

Dans d'autres conditions préférentielles de mise en oeuvre, on retient une composition pharmaceutique vaccinale ci-dessus, caractérisée en ce qu'elle renfermant renferme un adjuvant adsorbant le principe actif, tels l'hydroxyde d'alumine ou les particules d'or.

Dans encore d'autres conditions préférentielles de mise en oeuvre, on retient une composition pharmaceutique ci-dessus, caractérisée en ce que la protéine est obtenue par recombinaison génétique et présente une homologie peptidique de 70 % au moins avec la protéine Tat du HIV-1, ou avec un segment de cette protéine.

Dans toujours d'autres conditions préférentielles de mise en oeuvre, on retient une composition pharmaceutique ci-dessus, caractérisée en ce que la protéine a été traitée par un aldéhyde a été carboxyméthylée, carboxamidée ou maléimidée.

La présente invention a encore pour objet un procédé de préparation d'une composition ci-dessus décrite, caractérisé en ce que l'on mélange, selon des méthodes connues en elles mêmes, le ou les principes actifs avec des excipients acceptables, notamment pharmaceutiquement acceptables et le cas échéant, avec un adjuvant d'immunité mucosale.

Dans des conditions préférentielles de mise en oeuvre du procédé ci-dessus, on prépare des microgranules bioadhésifs et gastro résistants pour la voie orale digestive renfermant les principes actifs immunogènes et le cas échéant les adjuvants.

L'administration à un patient de composés immunogènes selon l'invention par voie mucosale correspond à une immunothérapie active. Il peut être intéressant également de procéder à une immunothérapie passive, c'est à dire de fournir à un patient directement des anticorps de classe IgA dont il a besoin pour neutraliser les effets nocifs des protéines ci-dessus, par exemple des protéines immunosuppressives à action locale induites par des tumeurs.

Ces anticorps de classe IgA par exemple anti-protéines immunosuppressives et/ou angiogéniques peuvent être obtenus classiquement.

C'est pourquoi la présente demande a également pour objet de tels procédés de préparation d'anticorps de classe IgA anti-protéines notamment immunosuppressives et/ou angiogéniques d'une tumeur cancéreuse et en particulier d'anticorps anti-protéine E7 du papilloma virus ou anti-protéine Tax de HTLV1, et notamment un procédé de préparation d'un anticorps de classe IgA ci-dessus caractérisé en ce que l'on immunise un mammifère à l'aide d'un composé immunogène tel que défini ci-dessus, puis récupère les anticorps formés.

La présente demande a encore pour objet un anticorps de classe IgA anti-protéine immunosuppressive ou angiogénique sécrété par les cellules d'une tumeur cancéreuse ou infectées par un virus tel le VIH-1 et en particulier des anticorps polyclonaux ou monoclonaux obtenus à partir de mammifères immunisés avec un composé immunogène défini ci-dessus et notamment une protéine immunosuppressive ou angiogénique d'une tumeur cancéreuse biologiquement inactivée mais immunogène, notamment la protéine E7 du papilloma virus ou la protéine Tax de HTLV1, ou leurs fragments. Ces anticorps administrés passivement, qu'ils soient allogéniques (humains) ou xénogéniques (animaux), pourront être des anticorps monoclonaux ou polyclonaux complets ou des fragments F(ab')2 ou Fab de l'anticorps.

Par "anticorps anti-protéine immunosuppressive ou angiogénique d'une tumeur cancéreuse", l'on entend des anticorps monoclonaux ou polyclonaux ou des fragments F(ab')2 ou Fab de ces anticorps ou encore des anticorps anti-protéine immunosuppressive ou angiogénique produits par les cellules d'une tumeur cancéreuse ou infectées par le VIH-1, obtenus par construction génétique à partir d'une librairie de phages.

Les anticorps xénogéniques proviennent d'animaux hyperimmunisés avec un composé immunogène selon l'invention, notamment avec la protéine E7 du papilloma virus ou la protéine Tax de HTLV1 ou ses dérivés (fragments peptidiques de la protéine E7 du papilloma virus ou de la protéine Tax de HTLV1 détoxifiés selon l'invention), et sont
- soit polyclonaux provenant d'animaux hyperimmunisés,
- soit monoclonaux, obtenus après hybridation selon la technique que Kohler et Milstein de cellules spléniques ou d'adénocytes avec une lignée myélomateuse, type x63, notamment x63AG3. Dans ce cas on préfère les anticorps équins ou de lapin.

La présente demande a encore pour objet un procédé de préparation d'anticorps de classe IgA anti-protéine immunosuppressive ou angiogénique d'une tumeur cancéreuse, caractérisé en ce que l'on immunise par voie muqueuse un mammifère, homme ou animal, avec un composé immunogène tel que défini ci-dessus.

La présente demande a également pour objet un procédé d'obtention d'anticorps de classe IgA anti-protéine immunosuppressive ou angiogénique, par la technologie de recombinaison génétique, caractérisé en ce que l'on utilise à titre d'immunogène un composé immunogène tel que défini ci-dessus.

La présente demande a encore pour objet des fragments F(ab')2 ou Fab desdits anticorps de classe IgA ; ceux-ci peuvent être obtenus par digestion enzymatique par exemple.

La présente invention a tout autant pour objet un procédé d'immunisation passive de sujets cancéreux ou malade du Sida, utilisant des anticorps spécifiques de classe IgA anti-protéine immunosuppressive ou angiogénique d'une tumeur cancéreuse ou produite par des cellules infectées par un virus et spécialement anti-protéine E7 du papilloma virus ou anti-protéine Tax de HTLV1 neutralisant ou bloquant les effets nocifs de cette protéine et pouvant être préparés comme indiqué ci-dessus, ou des fragments F(ab')2 ou F(ab) de ces anticorps.

La présente demande a également pour objet un procédé d'immunisation active caractérisé en ce que l'on utilise à titre d'immunogène un composé immunogène tel que défini ci-dessus avantageusement associé à un adjuvant d'immunité minéral, huileux ou de synthèse, ou encore un composé immunogène tel que défini ci-dessus, avantageusement couplé par exemple à l'aide d'un dialdéhyde ou associé à une protéine augmentant son immunogénicité ou encore une molécule d'ADN correspondant à la protéine à combattre mais inactivée par mutation.

Ces immunisations peuvent être réalisées tant à titre curatif qu'à titre préventif.

A titre d'immunogènes, pour tous les procédés ci-dessus et ci-après, on utilise de préférence un dérivé de la protéine E7 du papilloma virus ou de la protéine Tax de HTLV1.

L'invention a en outre pour objet une composition pharmaceutique pour la voie mucosale comprenant, à titre de principe actif curatif ou préventif , au moins un anticorps de classe IgA anti-protéine immunosuppressive ou angiogénique d'une tumeur cancéreuse tel que défini ci-dessus ou obtenu selon les procédés ci-dessus éventuellement associé à un anticorps de classe IgG anti-protéine immunosuppressive ou angiogénique d'une tumeur cancéreuse.

En résumé et notamment, la présente invention a pour objet l'usage à titre préventif, ou curatif chez le sujet cancéreux ou atteints de Sida d'anticorps de classe IgA de manière à bloquer l'action notamment immunosuppressive d'une protéine à action immunosuppressive locale ou angiogénique d'une tumeur cancéreuse ou des cellules infectées par un virus et notamment de la protéine Tat du VIH-1. Ces anticorps spécifiques de classe IgA pourront provenir :
1. du sujet lui-même, induits par une immunisation active (vaccination) avec une protéine immunosuppressive locale d'une tumeur cancéreuse sécrétée par les cellules infectées par un virus privée desdits effets immunosuppressifs mais immunogène (on a préservé les propriétés susceptibles d'induire la formation des anticorps, lorsque la protéine est présentée et préparée de manière appropriée, couplée ou non à un "carrier", agrégée ou non, en présence ou non d'adjuvant) ou avec une molécule d'ADN correspondant à la protéine à combattre mais inactivée par mutation ou audit fragment inactif ou
2. d'un organisme étranger, allo- ou xénogénique, administré au sujet par immunisation passive (sérothérapie). Ces anticorps de classe IgA administrés passivement, pourront être des anticorps monoclonaux ou polyclonaux complets ou des fragments F(ab')2 ou Fab de l'anticorps.

L'invention a également pour objet des compositions pharmaceutiques et notamment une composition pharmaceutique comprenant à titre d'agent préventif ou curatif des anticorps de classe IgA anti-protéine à action immunosuppressive ou angiogénique locale d'une tumeur cancéreuse produits à partir d'organismes immunisés contre ladite protéine ou ses fragments F(ab')2 ou Fab, selon l'invention.

En outre l'invention propose également un kit comprenant une composition pharmaceutique vaccinale qui en plus du principe actif (protéine initialement immunosuppressive ou angiogénique locale d'une tumeur cancéreuse mais privée desdits effets immunosuppressifs ou angiogéniques et immunogène ou ses dérivés ou anticorps de classe IgA anti-protéine immunosuppressive ou angiogénique locale d'une tumeur cancéreuse ou molécule d'ADN) peut comprendre un adjuvant et/ou un autre immunogène à propriétés anticancéreuses.

Enfin l'invention propose une composition pharmaceutique adaptée à l'administration mucosale.

Pour diminuer la charge immunosuppressive produite par la tumeur et améliorer encore mieux la réponse immunitaire, on pourra associer à cette immunisation anti-suppressive une immunisation par voie générale par exemple obtenue par injection sous-cutanée, voire des moyens plus classiques visant à diminuer la taille de la tumeur comme la chimiothérapie, la radiothérapie, l'exérèse chirurgicale ou encore l'adjonction de gènes suppresseurs des tumeurs apportés par des techniques de la thérapie génique (ADN véhiculés par des vecteurs viraux, vecteurs lipidiques etc..) ou encore comme l'immunisation active contre des protéines sans action immunosuppressive ou angiogénique locale comme les MAGE ou des protéines de structure comme celles du papilloma virus.

On pourra aussi associer d'autres immunisations anti-suppressives et/ou angiogéniques par exemple en immunisant contre des cytokines ou des lectines susceptibles de servir de médiateur à l'action suppressive sur les cellules immunitaires ou associer des immunisations contre des antigènes tumoraux classiques (non immunosuppressifs ou angiogéniques) susceptibles d'augmenter la réponse immunitaire cellulaire particulièrement tueuse (cellules CTL ou cellules NK) dirigée contre les cellules tumorales ou infectées par des virus. L'avantage de ces associations est qu'elles permettront au système immunitaire de mieux répondre à l'immunisation anti-immunosuppressive et par conséquent de mieux se reconstituer.

Pour résumer, à l'immunisation mucosale contre un facteur immunosuppressif ou angiogénique paracrine présenté sous une forme inactive mais toujours immunogène, on pourra associer des méthodes plus classiques comme radiothérapie, chimiothérapie exérèse chirurgicale ou traitement par gènes suppresseurs ou encore des immunisations contre des cytokines ou des lectines produites par des cellules immunitaires (cellules T ou APC) médiant l'action immunosuppressive et/ou angiogénique ou contre des antigènes tumoraux.

En effet, dans certains cancers, même d'origine virale, des facteurs solubles d'origine cellulaire, telles les cytokines ou les lectines peuvent également jouer localement un rôle de médiateur de l'immunosuppression et/ou de l'angiogénèse au sein des tumeurs. C'est le cas de l'IFNα, cytokine immunosuppressive, surproduit localement au sein des tissus lymphoïdes infectés par le HIV dans la maladie Sida.

L'expérimentation in vitro sur des cellules mononucléées du sang (PBMC) infectées par le HIV-1 a montré que la protéine Tat était impliquée dans la surproduction par les APC de la cytokine IFNα immunosuppressive. De manière intéressante, la protéine E7 du HPV, tout comme la protéine Tat du HIV-1, induit la surproduction d'IFNα immunosuppressif par les APC.

En conséquence, on pourra également combattre l'échappement immunitaire et/ou l'angiogénèse des cancers, en induisant ou en administrant des anticorps dirigés spécifiquement contre les cytokines dont la surproduction est responsable d'immunopathogénèse, en particulier d'immunosuppression, tel l'IFNα et/ou de l'angiogénèse, tel le TNFα conformément à la demande de brevet internationale WO 92/225.

Ainsi les effets immunosuppressifs dus à la surproduction d'IFNα dans les cancers de la maladie Sida et du col utérin tout comme ceux dus à la surproduction de TGFB dans les gliomes viro-induits pourront être bloqués par des anticorps dirigés contre ces cytokines naturelles induits par une immunisation active (vaccination) utilisant des cytokinoïdes (cytokines modifiées biologiquement inactives mais immunogènes) comme vaccin. De tels anticorps pourront également être administrés passivement (immunisation passive).

Les exemples et expérimentations qui suivent illustrent la présente demande.

### Exemple 1. Préparation de Tat carboxamidé

Le Tat carboxamidé est un produit inactif mais immunogène qui a été préparé par carboxyamidation de la protéine Tat recombinante native. Une préparation de la protéine Tat a notamment été décrite dans WO-A-99/33872.

Le Tat toxoïde a été préparé de la façon suivante :
La protéine Tat native recombinante (en solution la plus concentrée possible) est dialysée 16 heures contre du tampon Tris, HCL 0,3 M, contenant de la guanidine 6M et du Dithiothréitol 10mM (rapport volume solution de Tat / volume tampon de dialyse : 1 / 20).

Après dialyse, la solution de Tat est recueillie et son volume mesuré. La solution est désaérée par percussion sous pression réduite. On ajoute alors une solution d'acide iodoacétamide 0,5 M désaéré par circulation d'un courant d'azote (dans la proportion de 28 µl d'acide iodoacétamide 0,5 M par ml de solution Tat).

Sous atmosphère d'azote, la réaction s'accomplit pendant 90 minutes, à 37°C, à l'abri de la lumière.

La réaction est alors bloquée par addition de β-mercaptoéthanol concentré (0,65 µl par ml de mélange réactionnel).

Le mélange est à nouveau placé pendant 60 minutes, à 37°C, à l'abri de la lumière, sous atmosphère d'azote.

Le mélange est ensuite dialysé, sous agitation, contre du tampon Tris, HCL 0,3 M contenant :
- Urée 8 M : 2 H à température ambiante
- Urée 4 M : 2 H à température ambiante
- Urée 2 M : 2 H à température ambiante
- PBS 1X : 16 heures à 4°C.

La réaction de carboxyamidation permet de modifier les groupements thiols (groupements sulfhydryl) présents au niveau des résidus cystéines de l'enchaînement en acides aminés. Ces groupements réagissent avec l'acide iodoacétamide par une réaction de S-carboxyamidométhylation. Le produit de la réaction est un résidu S-carboxyméthylamidocystéinyl.

### Exemple 2. Préparation Vaccine recombinante (souche Lister) exprimant la gp160 du VIH-1 (Souche (LAV/IIIB) : construit donné par G. Beaud (Institut Jacques Monod, CNRS, Paris, France).

### Exemple 3. Plasmide d'expression pSV-Tat

Le plasmide d'expression pSV-Tat a été amplifié dans E. Coli et purifié par centrifugation en chlorure de césium (Advanced Biotechnologies Inc.).

Les composés immunogènes des exemples 1, 2 et 3 ont été associés à un adjuvant afin de potentialiser la réponse immunitaire.

### Les adjuvants

Différents types d'adjuvant ont été testés :
- la toxine thermolabile mutée (L.T mutée) de Escherichia coli entérotoxigène (ETEC) : la LT (R192G) décrite par Cardenas-Freytag L et al, Effectiveness of a vaccine composed of heat-killed Candida albicans and a novel mucosal adjuvant, LT (R192G), against systemic candidiasis, Infect immun; 1999, 67 :826-33.
- la sous-unité B (CTB) de la toxine cholérique (CT) de la bactérie Vibro cholerae contaminée par la CT totale.
- le Montanide IMS 1113,
- le C92512,
- l'ISA 51. (SEPPIC).

### Exemple 4

On a préparé une solution intranasale répondant à la formule

| | |
|---|---|
| composé de l'exemple 2. | 10 mg |
| excipient q.s. pour une solution intranasale terminée à | 20 ml |
| (détail de l'excipient : Labrasol^{®}, chlorure de sodium, benzoate de sodium, eau pour préparations injectables). | |

### Exemple 5

On a préparé une solution intranasale répondant à la formule

| | |
|---|---|
| Tat carboxyméthylé décrit dans WO-A-99/33872 | 10 mg |
| excipient q.s. pour une solution intranasale terminée à | 20 ml |
| (détail de l'excipient : Labrasol^{®}, chlorure de sodium, benzoate de sodium, eau pour préparations injectables). | |

### Exemple 6

On a préparé des capsules orales renfermant chacune :

| | |
|---|---|
| Composé de l'exemple 1 . | 200 µg |
| Alhydrogel de Superfos | 20 µg |
| Excipient per os | qsp. 0,5 ml |

### Etude pharmacologique

### Expérience 1.

Protocole : des souris ont reçu par voie intramusculaire 100 µl d'émulsion (1 :1), en ISA 51 contenant 25 µg de Tat carboxamidé de l'exemple 1 et 5 µg de LT au jour 0. Ensuite, aux jours 7, 14 et 21, ces souris ont reçu par voie intranasale 10 à 15 µl d'une préparation contenant 25 µg de Tat carboxamidé de l'exemple 1 et 3 µg de LT. Le sérum de ces souris a été prélevé au jour 28.

La recherche d'anticorps anti-Tat, de type IgG et IgA dans leur sérum a été réalisée par le test ELISA suivant : des plaques sont sensibilisées avec du de Tat carboxamidé (1 µg / puits). Les sérums, dilués au 1/4 pour détecter les anticorps de type IgA ou dilués au 1/50 pour les anticorps de type IgG, sont testés selon les protocoles standard d'ELISA. Les résultats obtenus sont exprimés en D.O.

| | Classe | Souris Contrôles | Souris immunisées |
|---|---|---|---|
| Ac. Anti-Tat | IgG | 0,175 | 2,224 |
| Ac. Anti-Tat | IgA | 0,461 | 1,759 |

Ce protocole d'immunisation a conduit à augmenter le taux des Anticorps spécifiques de classe IgG et IgA, assurant une immunité à la fois systémique et mucosale, confirmée par la présence d'IgA sécrétoires dans les lavages des sécrétions intestinales réalisées selon la technique de Elson C.V. et al. (J. of Immunol Methods, 1984, 67 : 101-108).

### Expérience 2 :

Protocole : des souris ont reçu par voie intranasale 10 à 15 µl d'émulsion (1 :1), en IMS 1113 ou C92512, contenant 25 µg de Tat carboxamidé et 5 µg de LT au jour 0. Ensuite, aux jours 7 et 14, ces souris ont reçu par voie intranasale 10 à 15 µl d'émulsion (1 :1), respectivement en IMS 1113 ou en C92512, contenant 25 µg de Tat toxoïde et 3 µg de LT. Le sérum de ces souris a été prélevé au jour 21. La recherche d'anticorps anti-Tat, de type IgG et IgA dans leur sérum a été réalisée par le test ELISA décrit dans le protocole de l'expérience 1.

| | Classe | Souris Contrôles | Souris immunisées (C92512) | Souris immunisées (IMS 1113) |
|---|---|---|---|---|
| Ac. Anti-Tat | IgG | 0,2 | 2,220 | 1,543 |
| Ac. Anti-Tat | IgA | 0,7 | 0,7 | 1,092 |

L'utilisation d'adjuvant IMS 1113 (Seppic, France) a permis d'induire une immunité mucosale, caractérisé par l'amplification du taux d'anticorps anti-Tat de classe IgA. Par contre, l'adjonction d'adjuvant C92512 (Seppic, France) ne l'a pas permis.

### Expérience 3 :

Protocole : des souris ont reçu par voie intranasale 10 à 15 µl d'une préparation contenant 10 µg du plasmide de l'exemple 3, 5 µg/ml de CTB et 5 µg/ml de CT au jour 0. Ensuite, aux jours 7 et 14, ces souris ont reçu par voie intranasale 10 à 15 µl d'une préparation contenant 25 µg de Tat toxoïde et 5 µg/ml de CTB et 0,5 µg/ml de CT. Le sérum de ces souris a été prélevé au jour 21. La recherche d'anticorps anti-Tat, de type IgG et IgA dans leur sérum a été réalisée par le test ELISA décrit dans le protocole de l'expérience 1.

| | Classe | Souris Contrôles | Souris immunisées |
|---|---|---|---|
| Ac. Anti-Tat | IgG | 0,3 | 2,123 |
| Ac. Anti-Tat | IgA | 0,2 | 1,951 |

L'immunisation par voie intranasale au moyen de plasmide anti-Tat caractérisé par une augmentation d'IgA et confirmée par la présence d'IgA sécrétoires dans les sécrétions intestinales.

### Expérience 4 :

Protocole : des souris ont reçu par voie orale 200 µl d'une préparation contenant 10⁶ PPU/ml de vaccine recombinante exprimant la gp160, 5 µg/ml de CTB et 0,5 µg/ml de CT aux jours 0, 7 et 14. Le sérum de ces souris a été prélevé au jour 21.

La recherche d'anticorps anti-Tat, de type IgG et IgA dans leur sérum a été réalisée par le test ELISA suivant : des plaques sont sensibilisées avec de la gp160 recombinante native (1µg/puits). Les sérums, dilués au 1/4 pour détecter les anticorps de type IgA ou dilués au 1/50 pour les anticorps de type IgG, sont testés selon les protocoles standards d'ELISA. Les résultats obtenus sont exprimés en D.O.

| | Classe | Souris Contrôles | Souris immunisées |
|---|---|---|---|
| Ac. Anti-gp160 | IgG | 0,112 | 1,283 |
| Ac. Anti-gp160 | IgA | 0,440 | 1,02 |

L'augmentation des anticorps de classe IgG et IgA reflète une immunité à la fois systémique et mucosale anti-gp160.

### Expérience 5 :

Protocole : des souris ont reçu par voie intranasale aux jours 0, 7, 14 et 21 10-15 µl de PBS contenant comme immunogène 50 µg de Tat carboxamidé de l'exemple 1 et comme adjuvant LT (3-5 µg) et 2 µl d'hydroxyde d'aluminium (Alhydrogel 85 de Superfos Biovector, Danemark). La recherche d'anticorps anti-Tat de type IgG et IgA dans leurs sérums a été réalisée par ELISA suivant le protocole de l'exemple 1.

| | Classe | Souris Contrôles | Souris immunisées |
|---|---|---|---|
| Ac. Anti-Tat | IgG | 0,182 | 2,042 |
| ²Ac. Anti-Tat | IgA | 0,461 | 1,258 |

L'agrégation de l'immunogène Tat Toxoïde autour des particules d'hydroxyde d'alumine en présence du LT a facilité la réponse anticorps anti-Tat.

## Revendications

1. Utilisation, en combinaison avec un adjuvant d'immunité mucosale, de la protéine Tat du virus vIH1 dont les propriétés immunosuppressive et/ou angiogénique sont rendues inactives d'au moins 70%, par un traitement physique et/ou chimique, par recombinaison génétique ou encore par un conditionnement adjuvant, ledit traitement lui conservant la propriété d'être reconnue par des anticorps dirigés contre ladite protéine Tat, et lui conservant des propriétés immunogènes suffisantes pour générer des anticorps neutralisant ou bloquant ladite protéine Tat native, ou encore l'utilisation d'une molécule d'ADN correspondant à ladite protéine Tat inactivée par mutation ou d'une molécule d'ADN correspondant audit fragment inactif, pour l'obtention d'un médicament conférant une immunité mucosale fondée sur la sécrétion d'anticorps de classe IgA, pour le traitement des cancers liés aux infections par le virus VIH1.

2. Utilisation selon la revendication 1, **caractérisée en ce que** ledit médicament consiste en une composition pharmaceutique vaccinale pour la voie muqueuse.

3. Utilisation selon l'une des revendications 1 et 2, **caractérisée en ce que** ledit médicament est adapté pour l'administration par voie orale ou par voie intranasale.

4. Utilisation selon l'une des revendications 1, 2 et 3, **caractérisée en ce que** le composé immunogène a été traité par un aldéhyde, ou carboxamidé, ou carboxyméthylé ou maléimidé, ou oxydé par barbotage d'oxygène.

5. Utilisation selon l'une des revendications 1, 2 et 3, **caractérisée en ce que** la protéine Tat utilisée est une protéine Tat mutée.

6. Utilisation selon l'une des revendications 1, 2 et 3, **caractérisée en ce que** l'on utilise une molécule d'ADN correspondant à ladite protéine Tat inactivée par mutation, ou une molécule d'ADN correspondant audit fragment inactif.

7. Une composition pharmaceutique vaccinale pour la voie muqueuse, renfermant à titre de principe actif la protéine Tat du virus VIH1 dont les propriétés angiogéniques et/ou immunosuppressives sont rendues inactives d'au moins 70%, par un traitement physique et/ou chimique, par recombinaison génétique ou encore par un conditionnement adjuvant, ledit traitement lui conservant la propriété d'être reconnue par des anticorps dirigés contre ladite protéine, et lui conservant des propriétés immunogènes suffisantes pour générer des anticorps neutralisant ou bloquant ladite protéine native, ou encore une molécule d'ADN correspondant à ladite protéine Tat inactivée par mutation, ou une molécule d'ADN correspondant audit-fragment inactif, en combinaison avec un adjuvant d'immunité mucosale.

8. Une composition pharmaceutique vaccinale selon la revendication 7, **caractérisée en ce qu'**elle consiste en une composition pour la voie orale digestive ou pour la voie nasale.

9. Une composition selon selon l'une des revendications 7 et 8, **caractérisée en ce que** l'adjuvant d'immunité mucosale est choisi parmi un mutant de CT (choléra toxine) et un mutant de LT (entérotoxine labile de *E*. *coli*).

10. Une composition pharmaceutique vaccinale selon l'une des revendications 7 à 9, **caractérisée en ce qu'**elle renferme un adjuvant absorbant le principe actif.

11. Une composition pharmaceutique vaccinale selon la revendication 10, **caractérisée en ce que** l'adjuvant adsorbant le principe actif est choisi parmi l'hydroxyde d'alumine et les particules d'or.

12. Une composition pharmaceutique selon l'une des revendications 7 à 11, **caractérisé en ce que** la protéine est obtenue par recombinaison génétique et présente une homologie peptidique de 70% au moins avec la protéine Tat du VIH1 ou avec un segment de la protéine Tat du VIH1.

13. Une composition pharmaceutique selon l'une des revendications 7 à 12, **caractérisée en ce que** la protéine a été traitée par un aldéhyde ou a été carboxyméthylée, carboxamidée ou maléimidée, ou oxydée par barbotage d'oxygène.

14. Procédé de préparation d'une composition pharmaceutique, **caractérisé en ce que** l'on prépare une composition pour la voie muqueuse, telle que définie à l'une des revendications 7 à 13, par mélange du ou des principes actifs immunogènes avec un adjuvant de l'immunité mucosale.

15. Un anticorps purifié de classe IgA anti-protéine Tat du virus VIH1.

16. Un fragment F(ab')₂ ou F(ab) d'un anticorps tel que défini à la revendication 15.

17. Une composition pharmaceutique renfermant un anticorps de classe IgA anti-protéine Tat selon l'une des revendications 15 ou 16.

18. Une composition pharmaceutique renfermant un anticorps de classe IgG anti-protéine Tat du virus VIH1 et un anticorps de classe IgA anti-protéine Tat du virus VIH1.

## Patentansprüche

1. Verwendung des Tat-Proteins des Virus HIV-1, dessen immunsuppressive und/oder angiogene Eigenschaft durch eine physikalische und/oder chemische Behandlung, durch genetische Rekombination oder auch durch eine Adjuvanskonditionierung um wenigstens 70% inaktiviert wurden, wobei die Behandlung dessen Eigenschaft, von gegen das Tat-Protein gerichteten Antikörpern erkannt zu werden, aufrechterhält und dessen zur Erzeugung von das native Tat-Protein neutralisierenden oder blockierenden Antikörpern ausreichenden immunogenen Eigenschaften aufrechterhält, oder auch Verwendung eines DNA-Moleküls, das dem durch Mutation inaktivierten Tat-Protein entspricht, oder eines DNA-Moleküls, das dem inaktiven Fragment entspricht, in Kombination mit einem Schleimhautimmunitätsadjuvans zur Gewinnung eines Medikaments, das eine Schleimhautimmunität auf der Grundlage der Sekretion von Antikörpern der Klasse IgA verleiht, für die Behandlung von Krebserkrankungen, die mit Infektionen durch das Virus HIV-1 zusammenhängen.

2. Verwendung gemäß Anspruch 1, **dadurch gekennzeichnet, dass** das Medikament aus einer pharmazeutischen Impfzusammensetzung zur Verabreichung über die Schleimhaut besteht.

3. Verwendung gemäß einem der Ansprüche 1 und 2, **dadurch gekennzeichnet, dass** das Medikament für die orale oder intranasale Verabreichung geeignet ist.

4. Verwendung gemäß einem der Ansprüche 1, 2 und 3, **dadurch gekennzeichnet, dass** die immunogene Verbindung mit einem Aldehyd behandelt oder carboxamidiert oder carboxymethyliert oder maleinimidiert oder mittels Durchperlenlassen von Sauerstoff oxidiert wurde.

5. Verwendung gemäß einem der Ansprüche 1, 2 und 3, **dadurch gekennzeichnet, dass** das verwendete Tat-Protein ein mutiertes Tat-Protein ist.

6. Verwendung gemäß einem der Ansprüche 1, 2 und 3, **dadurch gekennzeichnet, dass** ein DNA-Molekül, das dem durch Mutation inaktivierten Tat-Protein entspricht, oder ein DNA-Molekül, das dem inaktiven Fragment entspricht, verwendet wird.

7. Pharmazeutische Impfzusammensetzung zur Verabreichung über die Schleimhaut, die als Wirkstoff das Tat-Protein des Virus HIV-1, dessen immunsuppressive und/oder angiogene Eigenschaft durch eine physikalische und/oder chemische Behandlung, durch genetische Rekombination oder auch durch eine Adjuvanskonditionierung um wenigstens 70% inaktiviert wurden, wobei die Behandlung dessen Eigenschaft, von gegen das Tat-Protein gerichteten Antikörpern erkannt zu werden, aufrechterhält und dessen zur Erzeugung von das native Tat-Protein neutralisierenden oder blockierenden Antikörpern ausreichenden immunogenen Eigenschaften aufrechterhält, oder auch Verwendung eines DNA-Moleküls, das dem durch Mutation inaktivierten Tat-Protein entspricht, oder eines DNA-Moleküls, das dem inaktiven Fragment entspricht, in Kombination mit einem Schleimhautimmunitätsadjuvans umfasst.

8. Pharmazeutische Impfzusammensetzung gemäß Anspruch 7, **dadurch gekennzeichnet, dass** sie aus einer Zusammensetzung für die orale digestive Verabreichung oder für die nasale Verabreichung besteht.

9. Zusammensetzung gemäß einem der Ansprüche 7 und 8, **dadurch gekennzeichnet, dass** das Schleimhautimmunitätsadjuvans aus einer Mutanten des CT (Choleratoxins) oder einer Mutanten des LT (labilen Enterotoxins von *E*. *coli*) ausgewählt ist.

10. Pharmazeutische Impfzusammensetzung gemäß einem der Ansprüche 7 bis 9, **dadurch gekennzeichnet, dass** sie ein Adjuvans umfasst, das den Wirkstoff adsorbiert.

11. Pharmazeutische Impfzusammensetzung gemäß Anspruch 10, **dadurch gekennzeichnet, dass** das Adjuvans, das den Wirkstoff adsorbiert, aus Aluminiumhydroxid und Goldteilchen ausgewählt ist.

12. Pharmazeutische Zusammensetzung gemäß einem der Ansprüche 7 bis 11, **dadurch gekennzeichnet, dass** das Protein durch genetische Rekombination erhalten ist und eine Peptidhomologie von wenigstens 70% mit dem Tat-Protein von HIV-1 oder mit einem Segment des Tat-Proteins von HIV-1 aufweist.

13. Pharmazeutische Zusammensetzung gemäß einem der Ansprüche 7 bis 12, **dadurch gekennzeichnet, dass** das Protein mit einem Aldehyd behandelt oder carboxymethyliert, carboxamidiert oder maleinimidiert oder mittels Durchperlenlassen von Sauerstoff oxidiert wurde.

14. Verfahren zur Herstellung einer pharmazeutischen Zusammensetzung, **dadurch gekennzeichnet, dass** eine Zusammensetzung zur Verabreichung über die Schleimhaut gemäß einem der Ansprüche 7 bis 13 durch Mischen des immunogenen Wirkstoffs oder der immunogenen Wirkstoffe mit einem Schleimhautimmunitätsadjuvans hergestellt wird.

15. Gereinigter Antikörper der Klasse IgA, der gegen das Tat-Protein des Virus HIV-1 gerichtet ist.

16. F(ab')₂- oder F(ab)-Fragment eines Antikörpers gemäß Anspruch 15.

17. Pharmazeutische Zusammensetzung, die einen Antikörper der Klasse IgA, der gegen das Tat-Protein gerichtet ist, gemäß einem der Ansprüche 15 oder 16 umfasst.

18. Pharmazeutische Zusammensetzung, die einen Antikörper der Klasse IgG, der gegen das Tat-Protein des Virus HIV-1 gerichtet ist, und einen Antikörper der Klasse IgA, der gegen das Tat-Protein des Virus HIV-1 gerichtet ist, umfasst.

## Claims

1. Use, in combination with an adjuvant of mucosal immunity, of the HIV-1 virus Tat protein which immunosuppressive and/or angiogenic properties are inactivated by at least 70%, by a physical and/or chemical treatment, by genetic recombination or also by adjuvant conditioning, said treatment conserving its property of being recognized by antibodies directed against said Tat protein, and conserving sufficient immunogenic properties to generate antibodies neutralizing or blocking said native Tat protein, or also the use of a DNA molecule corresponding to said Tat protein inactivated by mutation or of a DNA molecule corresponding to said inactive fragment, for obtaining a medicament intended to give to a patient mucosal immunity based on the secretion of IgA class antibodies, for the treatment of cancers related to infections by HIV-1 virus.

2. Use according to claim 1, **characterized in that** the said medicament is a vaccinal pharmaceutical composition for the mucous route

3. Use according to one of claims 1 and 2 **characterized in that** the said medicament is adapted for administration by oral route or by intranasal route.

4. Use according to one of claims 1, 2 and 3 **characterized in that** the immunogenic compound was treated with an aldehyde, or carboxamidated, or carboxymethylated or maleimidated, or oxidized by oxygen bubbling.

5. Use according to one of claims 1, 2 and 3 **characterized in that** the Tat protein used is a mutated Tat protein.

6. Use according to one of claims 1, 2 and 3, **characterized in that** a DNA molecule corresponding to said Tat protein inactivated by mutation or a DNA molecule corresponding to said inactive fragment is used.

7. A pharmaceutical vaccinal composition for the mucous route, containing as active ingredient the HIV-1 virus Tat protein, which angiogenic and/or immunosuppressive properties are inactivated by at least 70%, by a physical and/or chemical treatment, by genetic recombination or also by adjuvant conditioning, said treatment conserving its property of being recognized by antibodies directed against said protein, and conserving sufficient immunogenic properties to generate antibodies neutralizing or blocking said native protein, or also a DNA molecule corresponding to said Tat protein inactivated by mutation or a DNA molecule corresponding to said inactive fragment, in combination with an adjuvant of mucosal immunity.

8. A pharmaceutical vaccinal composition according to claim 7, **characterized in that** it consists in a composition for the oral digestive route or for the nasal route.

9. A composition according to one of claims 7 and 8, **characterized in that** the adjuvant of mucosal immunity is chosen among a mutant of CT (cholera toxin) and a mutant of LT (*E*. *coli* labile enterotoxin).

10. A vaccinal pharmaceutical composition of vaccine according to one of claims 7 to 9 **characterized in that** it contains an adjuvant adsorbing the active ingredient.

11. A vaccinal pharmaceutical composition of vaccine according to claim 10 **characterized in that** the adjuvant adsorbing the active ingredient is chosen among aluminum hydroxide and gold particles.

12. A pharmaceutical composition according to one of claims 7 to 11 **characterized in that** the protein is obtained by genetic recombination and has a peptide homology of at least 70% with the HIV-1 Tat protein or with a segment of the HIV-1 Tat protein.

13. A pharmaceutical composition according to one of claims 7 to 12 **characterized in that** the protein was treated with an aldehyde, or was carboxamidated, or carboxymethylated or maleimidated, or oxidized by oxygen bubbling.

14. Process for the preparation of a pharmaceutical composition, **characterized in that** a composition is prepared for the mucous route, as defined in one of claims 7 to 13, by mixing the immunogenic active ingredient(s) with an adjuvant of mucosal immunity.

15. A purified IgA class antibody anti-HIV-1 virus Tat protein.

16. A F(ab')2 or F(ab) fragment of an antibody as defined in claim 15.

17. A pharmaceutical composition containing an IgA class anti-HIV-1 virus Tat protein according to one of claims 15 or 16.

18. A pharmaceutical composition containing an IgG class anti-HIV-1 virus Tat protein and an IgA class anti-HIV-1 virus Tat protein.
